# EUROPÄISCHE PATENTSCHRIFT

(11) **EP 2 306 943 B1**
(45) Veröffentlichungstag und Bekanntmachung des Hinweises auf die Patenterteilung: **25.01.2012**
(21) Anmeldenummer: 09775955.9
(22) Anmeldetag: 13.07.2009
(51) Int. Cl.: A61F 5/01, F16F 1/00

(54) **GELENKEINSATZ**
ARTICULATED INSERT
INSERT ARTICULAIRE

(30) Priorität: 24.07.2008 DE 102008034750
(43) Veröffentlichungstag der Anmeldung: 13.04.2011
(73) Patentinhaber: Otto Bock Healthcare GmbH, 37115 Duderstadt (DE)
(72) Erfinder: ANWAND, Gregor, 37115 Duderstadt (DE); HARDT, Alexander, 37115 Duderstadt (DE); KROLL-ORYWAHL, Olaf, 37085 Göttingen (DE); SCHIMEK, Norbert, 37339 Kirchworbis (DE); SEGL, Maximilian, 37115 Duderstadt (DE)
(74) Vertreter: Stornebel, Kai
(86) Internationale Anmeldenummer: PCT/DE2009/000970
(87) Internationale Veröffentlichungsnummer: WO 2010/009702

(56) Entgegenhaltungen:
- CH-A- 320 050
- FR-A- 2 827 158
- US-A- 4 669 457

## Beschreibung

Die Erfindung betrifft einen Gelenkeinsatz für eine orthopädische Einrichtung mit einem oberen Anschlussmittel zur Befestigung an einem ersten orthopädischen Bauteil und einem unteren Anschlussmittel zur Befestigung an einem zweiten, relativ zu dem ersten orthopädischen Bauteil verschwenkbar gelagerten orthopädischen Bauteil mit einem Verbindungselement zwischen den beiden Anschlussmitteln, das zugkraftübertragend ausgebildet ist. Der Gelenkeinsatz ist insbesondere für Orthesen vorgesehen, z. B. eine Sprunggelenksorthese.

Die EP 712 468 B1 beschreibt eine zusammengesetzte Biegungseinheit mit zwei Befestigungseinrichtungen zur Befestigung an zwei verschwenkbar zueinander angeordneten Orthesenbauteilen. Zwischen den beiden Befestigungseinrichtungen ist ein zugkraftübertragendes Lasten-Tragelement angeordnet, das aus einer Faser mit einem niedrigen Reibungskoeffizienten ausgebildet ist, wobei die Faser um Endmuffen gewickelt ist. Das Lasten-Tragelement ist in jede Richtung frei biegbar. Die Befestigungseinrichtungen und das Lasten-Tragelement sind in einem elastischen Kunststoff eingespritzt. Alternativ ist das Lasten-Tragelement als ein Gewebestreifen ausgebildet.

Weiterhin können kardanische Gelenkeinrichtungen oder Kettenverbindungen zwischen zwei orthopädietechnischen Elementen vorgesehen sein, um diese miteinander zu verbinden. Die kardanische Lagerung kann beispielsweise als ein Kugelgelenk oder eine klassische Kardanlagerung mit sich kreuzenden Achsen ausgebildet sein. Die Kettenverbindungen können über Kugelketten verwirklicht werden, die in korrespondierende Schalenlager eingelegt sind. Solche Lösungen können zu Luxationen der Gelenkkugeln führen und bieten insgesamt eine unbefriedigende Führung der Bauteile zueinander.

Die FR 2827158 A1 beschreibt eine Orthese mit einer Flachfeder im Fersenbereich.

Die US 4,669,457 beschreibt eine Ortheseneinrichtung mit einem Gelenk und

Manschetten zum Anlegen an den Oberschenkel und den Unterschenkel. Ein langgestrecktes Verbindungsteil ist an den Manschetten befestigt und ermöglicht eine Beugung in eine Richtung und steht einer Beugung in die entgegengesetzte Richtung entgegen.

Die CH 230050 B1, das als nächster stand der technik augesehen wird, eine gummielastische Zugfeder mit einer Einlage aus einem Gewebe oder Metall. Die Anlage aus Gewebe kann zwischen den Schlaufen genäht oder schlaufenbildend gewoben sein.

Aufgabe der vorliegenden Erfindung ist es, ein Gelenkeinsatz bereitzustellen, mit dem es möglich ist, zwischen zwei orthopädischen Elementen, insbesondere Orthesenelementen, eine flexible Verbindung mit federnder Wirkung zueinander zu ermöglichen, wobei eine Schwenkbewegung um eine oder mehrere definierte Achsen möglich sein soll. Erfindungsgemäß wird diese Aufgabe durch einen Gelenkeinsatz mit den Merkmalen des

Anspruchs 1 gelöst. Vorteilhafte Ausgestaltungen und Weiterbildungen der Erfindung sind in den Unteransprüchen ausgeführt.

Der erfindungsgemäße Gelenkeinsatz mit einem oberen Anschlussmittel zur Befestigung an einem ersten orthopädischen Bauteil und einem unteren Anschlussmittel zur Befestigung an einem zweiten, relativ zu dem ersten orthopädischen Bauteil verschwenkbar gelagerten orthopädischen Bauteil mit einem Verbindungselement zwischen den beiden Anschlussmitteln, das zugkraftübertragend ausgebildet ist, sieht vor, dass das Verbindungselement einen biegeelastischen, formstabilen Materialabschnitt aufweist, der einen Querschnitt mit unterschiedlichen axialen Trägheitsmomenten aufweist und die Achse des kleineren axialen Trägheitsmomentes zumindest abschnittsweise parallel zu der Schwenkachse der orthopädischen Bauteile liegt. Durch die Biegeelastizität des Materialabschnittes ist es möglich, eine Federwirkung bereitzustellen, so dass eine verbesserte Führung und Zuordnung der beiden orthopädischen Bauteile zueinander möglich ist. Im Falle eines Ortheseneinsatzes wird dadurch das zu unterstützende Körperglied besser geführt. Darüber hinaus wird eine flexible Verbindung der beiden orthopädischen Bauteile erreicht, ohne dass sich die Abstände zwischen den Verbindungsstellen bzw. Anschlussstellen wesentlich ändern, wodurch eine Relativbewegung der beiden orthopädischen Bauteile zueinander weitestgehend vermieden wird. Die Anordnung der Achsen der Flächenträgheitsmomente bewirkt, dass einerseits eine leichte und erwünschte Beugung oder Verlagerung der Bauteile um eine Achse ermöglicht wird, während ein Verbiegen um die zweite Achse aufgrund des höheren Flächenträgheitsmomentes und damit Biegewiderstandes wie gewünscht erschwert wird.

Die Erfindung sieht vor, dass der Materialabschnitt in seiner Längserstreckung in sich verdreht ist. Durch die wendelartige Gestaltung des flächigen Materialabschnittes ist es möglich, dass die jeweilige Biegeachse, die innerhalb des flächigen Materialabschnittes liegt, also parallel zu der Breite des Materialabschnittes verläuft, präzise eingestellt werden kann, insbesondere kann die Position der Biegeachse präzise bestimmt werden, indem die Verdrehung so ausgeführt wird, dass die Linie mit dem geringsten Flächenträgheitsmoment und mit dem geringsten Elastizitätsmodul dorthin gelegt wird, wo die Schwenkachse zwischen den beiden orthopädischen Bauteilen liegen soll.

Eine Weiterbildung der Erfindung sieht vor, dass der Materialabschnitt um zumindest 90° in sich verdreht ist, um eine Biegebewegung um zwei senkrecht zueinander orientierte Achsen zu ermöglichen. Bevorzugt ist der Materialabschnitt, der streifenförmig ausgebildet ist, um ganzzahlige Vielfache von 90° verdreht und insbesondere 180° oder 360°, um zwei oder drei senkrecht zueinander orientierte Biegeachsen bereitstellen zu können.

Bevorzugt besteht der Materialabschnitt aus Blech oder Kunststoff, wobei die Materialwahl so getroffen werden sollte, dass eine ausreichende Elastizität und Rückfederwirkung gegeben ist. Ebenfalls muss die Dauerbiegefestigkeit des Materialabschnittes bzw. des gesamten Verbindungselementes gegeben sein, damit der Gelenkeinsatz dauerhaft zuverlässig arbeiten kann.

Eine Weiterbildung der Erfindung sieht vor, dass das Verbindungselement zwei separate Materialabschnitte mit daran angeordneten Formschlusselementen aufweist, die zugkraftübertragend ineinander eingreifen und eine Verschwenkbarkeit um eine Achse zulassen. Durch die Verschwenkbarkeit um eine Achse ist es möglich, eine mehr oder weniger freie Beweglichkeit der beiden Anschlussmittel und damit der orthopädischen Einrichtungen, wie z. B. Orthesenbauteile bereitzustellen, ohne die Rückstellkraft aufgrund der Federwirkung der Materialabschnitte berücksichtigen zu müssen. Dies kann für besondere Einsatzzwecke günstig sein, wenn eine Beweglichkeit um eine Achse frei möglich sein soll, beispielsweise um eine Vorzugsbewegungsrichtung anzugeben, während andere Bewegungsrichtungen gegen einen Biegewiderstand ausgeführt werden müssen.

Der Materialabschnitt ist bevorzugt einlagig ausgebildet, kann jedoch auch aus einem Laminat oder einem Kompositbauteil bestehen. Alternativ zu einer einlagigen Ausbildung des Materialabschnittes ist vorgesehen, dass dieser aus einer geschlossenen Schlaufe ausgebildet ist und eine entsprechende Formgebung aufweist, so dass die Verbindung zwischen den beiden Anschussmitteln bei gleichzeitiger Bereitstellung einer ausreichenden Biegeelastizität gewährleistet ist.

Ebenfalls kann es möglich sein, dass das gesamte Verbindungselement als eine geschlossene Schlaufe mit einander gegenüberliegenden Materialabschnitten ausgebildet ist, so dass das Verbindungselement insgesamt aus einem Rohrabschnitt oder Schlauchabschnitt, z. B. bei der Ausgestaltung des Verbindungselementes als ein faserverstärktes Kunststoffbauteil, möglich ist.

Die Anschlussmittel weisen bevorzugt eine Hülse auf, die Schrauben oder ähnliche Befestigungsmittel aufnehmen, so dass eine einfache Befestigung der Anschlussmittel an den jeweiligen orthopädischen Einrichtungen möglich ist. Die Hülse kann einstückig an dem Verbindungselement und insbesondere einstückig mit dem Materialabschnitt als ein Umformabschnitt, als eine Wicklung oder als ein Schlaufenendabschnitt ausgebildet sein, so dass keine zusätzlichen Fügeverrichtungen durchgeführt werden müssen. Alternativ können sowohl die Hülse als auch die Anschlussmittel separat an dem Verbindungselement befestigt sein, beispielsweise um hohe Druckstabilitäten bei der Befestigung über eine separate Materialwahl realisieren zu können.

Da das Verbindungselement insbesondere bei einer Ausgestaltung als Blech oder GFK-Bauteil scharfkantig sein kann, ist vorgesehen, dass dieses ummantelt ausgebildet ist, insbesondere von einem flexiblem Elastomer, das neben einer Schutzfunktion für das Verbindungselement und die Anschlussmittel gegen äußere Einflüsse auch einen Kontaktschutz für den Träger der orthopädischen Einrichtung bereitstellt und darüber hinaus die Handhabbarkeit verbessert. Zusätzlich wird durch das Elastomer ein zusätzlicher Anteil an Rückstellkraft aufgebracht, zusätzlich zu der Biegeelastizität durch das Verbindungselement bzw. den Materialabschnitt.

Nachfolgend werden Ausführungsbeispiele der Erfindung anhand der beigefügten Figuren näher erläutert. Es zeigen:
- Figuren 1 a und 1b -: Draufsicht und Seitenansicht einer ersten Variante;
- Figuren 2a und 2b -: eine zweite Variante mit einstückig ausgebildeten Anschlussmitteln;
- Figuren 3a und 3b -: eine zweiteilige Variante des Gelenkeinsatzes mit zwei Verbindungselementen; sowie
- Figuren 4a bis 4c -: eine weitere Variante des Gelenkeinsatzes.

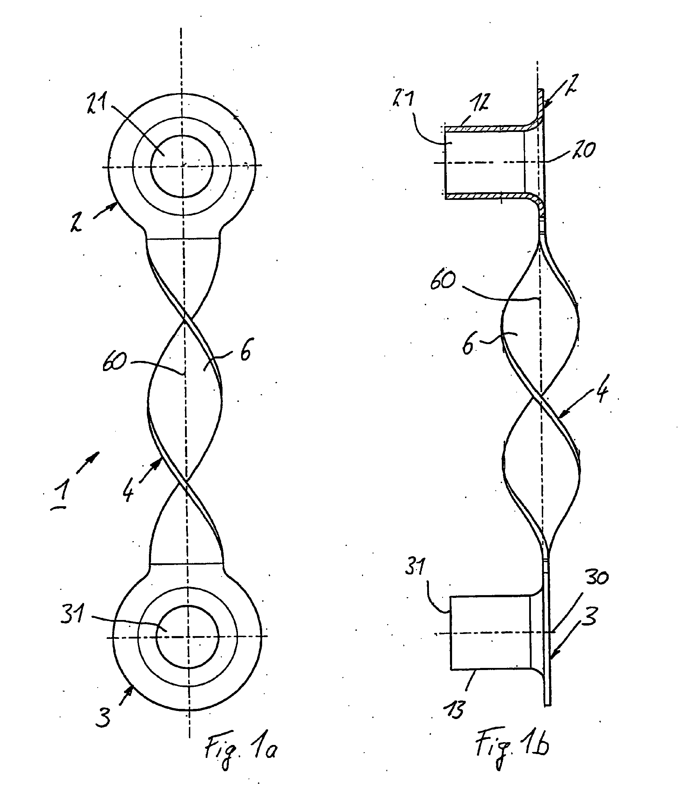

Figur 1a zeigt in Draufsicht einen Gelenkeinsatz 1 mit einem oberen Anschlussmittel 2 und einem unteren Anschlussmittel 3 zur Befestigung an zwei nicht dargestellten orthopädischen Bauteilen, beispielsweise Orthesenkomponenten. Das untere Anschlussmittel 3 kann beispielsweise an einem Fußteil einer Sprunggelenksorthese befestigt werden, während das obere Anschlussmittel 2 an dem unterschenkelseitigen Element der Sprunggelenksorthese befestigt ist. Die Befestigung erfolgt bevorzugt über Schrauben, die durch Öffnungen 21, 31 innerhalb der Anschlussmittel 2, 3 hindurch geführt werden. An den Anschlussmitteln 2, 3 sind Hülsen 12, 13 ausgebildet, die in der Seitenansicht gemäß Figur 1b zu erkennen sind. Die Hülsen 12, 13 sind einstückig mit den Anschlussmitteln 2, 3 ausgebildet und erstrecken sich im Wesentlichen senkrecht zu flächigen Flanschen, die als Auflagebereiche für Schrauben dienen können. Die Schrauben werden entlang der Mittelachsen 20, 30 der Hülsen 12, 13 eingeführt, so dass der Gelenkeinsatz 1 sicher und doch lösbar an den orthopädischen Bauteilen befestigt werden kann.

Zwischen den Anschlussmitteln 2, 3 ist ein Verbindungselement 4 ausgebildet, das aus einem wendelartig verdrehten Materialstreifen 6 besteht. Der Materialstreifen 6 ist um 360° entlang seiner Längserstreckung 60 verdreht und bildet eine Wendel aus. Der Materialstreifen 6, bevorzugt ein Blechstreifen, ist formstabil und gewährleistet einen definierten Abstand zwischen den beiden Anschlussmittel 2, 3. Der Materialstreifen 6 kann Zug- und Druckkräfte aufnehmen und ist biegeelastisch ausgebildet, so dass die beiden nicht dargestellten orthopädischen Bauteile in einem nicht angelegten Zustand stabil zueinander orientiert bleiben. Werden die beiden Anschlussstellen relativ zueinander bewegt, beispielsweise um die Mittelachsen 20, 30 verdreht, findet eine Biegung an zwei Stellen innerhalb des Materialstreifens 6 statt, nämlich dort, wo die Schmalseiten des Materialstreifens die Mittelachsen 60 schneiden. Eine Biegung findet also am oberen Viertel und unteren Viertel des Materialstreifens 6 statt.

In Bezugnahme auf Figur 1b findet eine Biegung senkrecht zu der Längserstreckung 6 in der Mitte zwischen den beiden Anschlussmitteln 2, 3 statt, auch wieder dort, wo die Schmalseite des Materialstreifens 6 die Mittelachse 60 schneidet, weil dort das geringste Flächenträgheitsmoment und das geringste Elastizitätsmodul vorhanden ist. Auf diese Art und Weise ist es möglich, einen federnden Gelenkeinsatz 1 bereitzustellen, der in der Ausführungsform gemäß den Figuren 1 a und 1b einstückig aus einem Blechbauteil hergestellt ist. Die Hülsen 12, 13 können im Rahmen eines Druckumformverfahrens, ähnlich einem Tiefziehverfahren, ausgebildet werden. Anschließend werden die beiden Anschlussmittel 2, 3 um 360° zueinander verdreht, so dass eine wendelartige Ausgestaltung des Verbindungselementes 1 erreicht wird. Die Federwirkung wird durch die Materialbeschaffenheit erreicht, die Positionierung der jeweiligen bevorzugten Schwenkachsen erfolgt durch die Anordnung und Ausgestaltung des Wendelverlaufs des Materialabschnittes 6, wobei bevorzugt Schwenkachsen vorgesehen sind, die parallel zu den Mittelachsen 20, 30 der Hülsen 12, 13 verlaufen, wie in Figur 1a gezeigt, sowie senkrecht dazu, wie in der Figur 1b zu erkennen ist.

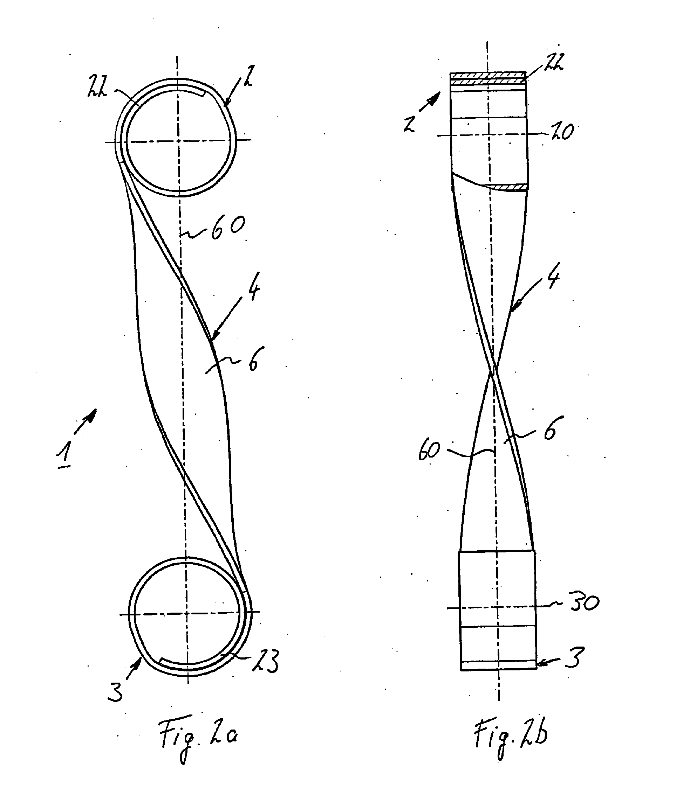

In den Figuren 2a und 2b ist eine Variante der Erfindung dargestellt, bei der ebenfalls ein Gelenkeinsatz 1 dargestellt ist, der aus einem einzigen Materialstreifen 6, vorliegend einem Blechstreifen, ausgebildet ist. Die Anschlussmittel 2, 3 sind durch Aufwickeln von Endbereichen ausgebildet, so dass entsprechende Hülsen 22, 23 zur Aufnahme von Scharuben oder anderen Befestigungselementen ausgebildet werden, die hinreichend formstabil sind. Der Materialstreifen 6 ist ebenfalls wendelförmig ausgebildet, vorliegend um 180° verdreht, so dass der Materialstreifen 6 tangentenartig an den Hülsen 22, 23 auf unterschiedlichen Seiten der Mittellinie 60 anliegt. Eine bevorzugte Biegung erfolgt um eine Biegeachse senkrecht zur Blattebene, wie in der Figur 2b gezeigt ist, an der Stelle, an der die Schmalseite des Materialstreifens 6 die Mittellinie 60 schneidet. Eine Verschwenkbarkeit um eine Schwenkachse parallel zu den Mittelachsen 20, 30 der Hülsen 22, 23 ist nur mit einem wesentlich höheren Kraftaufwand möglich, da der Materialstreifen 6 nicht entsprechend orientiert ist.

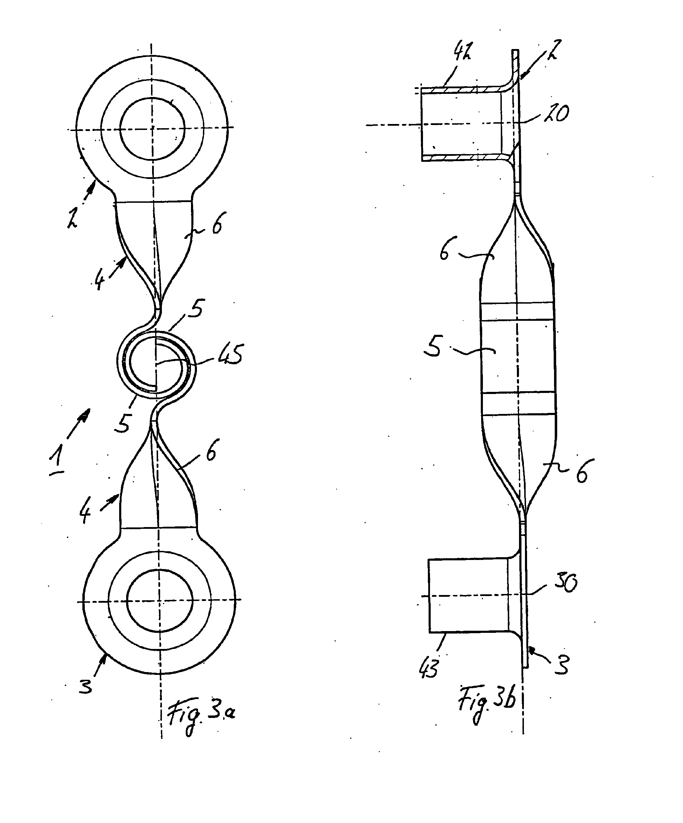

Eine weitere Variante ist in den Figuren 3a, 3b gezeigt, bei der der Gelenkeinsatz 1 zweiteilig ausgebildet ist. Dabei sind zwei identisch ausgebildete Blechbauteile vorgesehen, die jeweils ein Anschlussmittel 2, 3 und ein Verbindungselement 4 in Gestalt eines angeformten Materialstreifens 6 ausbilden. An dem Ende der Materialstreifen 6 sind Formschlusselemente 5 in Gestalt von hakenartigen, runden Biegeabschnitten ausgebildet, die so ausgebildet sind, dass die Breite des Materialstreifens 6 parallel zu den Hülsen 42, 43 orientiert sind. Dies erfolgt, indem der Materialstreifen 6 um 90° verdreht ist. Die Formschlusselemente 5 sind ineinander eingefügt und verschwenken aufgrund der Teilkreisausgestaltung der Formschlusselemente 5 um eine mittig zwischen den Anschlussmitteln 2, 3 angeordnete Schwenkachse 45. Die Schwenkachse 45 erstreckt sich senkrecht aus der Blattebene und verläuft parallel zu den Mittelachsen 20, 30 der Anschlussmittel 2, 3. Eine Biegeelastizität wird daher um diese Schwenkachse 45 nicht durch die Federsteifigkeit der Materialabschnitte 6 bereitgestellt, sondern gegebenenfalls durch andere elastische Mittel, die den Gelenkeinsatz 1 umgeben, beispielsweise umspritztes Silikon oder ein anderer Elastomer. Eine Biegeelastizität im Bereich der Materialabschnitte 6 erfolgt in der Nähe zum jeweiligen Übergang zu den Anschlussmitteln 2, 3 bzw. den Flanschbereichen der Anschussmittel 2, 3.

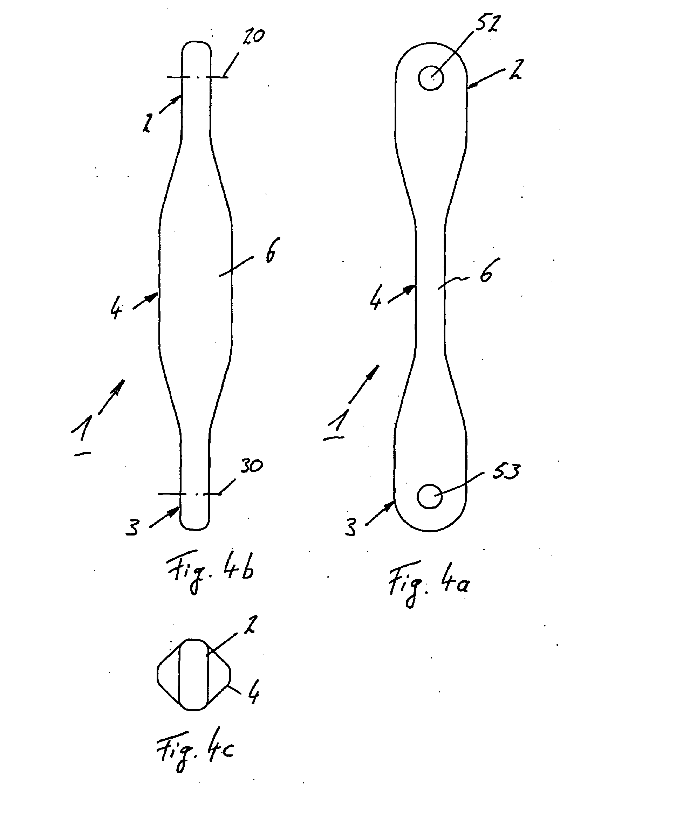

In den Figuren 4a bis 4c ist eine weitere Variante eines Gelenkeinsatzes 1 gezeigt. Der Gelenkeinsatz 1 weist obere und untere Anschlussmittel 2, 3 mit Ausnehmungen 52, 53 auf, die um Mittelachsen 20, 30 herum angeordnet sind. Die Figur 4a zeigt einen Gelenkeinsatz 1 in Seitenansicht, die Figur 4b zeigt einen Gelenkeinsatz 1 in Seitenansicht um 90° gedreht. Zwischen den beiden Anschlussmitteln 2, 3 ist das Verbindungselement 4 einstückig ausgebildet und weist einen flachen, im Querschnitt im Wesentlichen rechteckigen Materialabschnitt 6 in Streifenform auf, der zwischen den Anschlussmitteln 2, 3 in gleichbleibender Orientierung angeordnet ist. Aufgrund der rechteckigen Querschnittsform des Materialabschnittes 6 ergeben sich unterschiedliche große axiale Flächenträgheitsmomente und somit unterschiedlich große Biegewiderstände. In der Figur 4 ist die Orientierung des Materialabschnittes 6 so gewählt, dass die Achse des niedrigeren Flächenträgheitsmomentes parallel zu den Mittelachsen 20, 30 der Anschlussmittel 2, 3 ausgerichtet ist, so dass bei einer üblichen Befestigung über Schrauben oder dergleichen parallel zur Bewegungsachse der beiden zu verbindenden orthopädischen Komponenten die Achse des kleinen Flächenträgheitsmomentes parallel zur Schwenkachse der Orthesenkomponenten verläuft.

In der Figur 4c ist der Gelenkeinsatz 1 in einer Draufsicht gezeigt. Hier ist zu erkennen, dass auch die Anschlussmittel 2 einen im Wesentlichen rechteckigen Querschnitt aufweisen, wobei die beiden Längsachsen der rechteckigen Querschnitte der Anschlussmittel 2, 3 und des Materialabschnittes 6 senkrecht zueinander ausgerichtet sind. Der Übergangsbereich zwischen den Aufnahmeeinrichtungen 2, 3 und dem Materialabschnitt 6 stellt einen kontinuierlichen Übergang zwischen den Querschnitten dar, alternativ können auch diskontinuierliche Übergänge zwischen den Anschlussmitteln 2, 3 und dem Materialabschnitt 6 erfolgen. Der Gelenkeinsatz 1 kann einstückig aus einem entsprechend elastischen Material hergestellt sein, beispielsweise einem Metall oder Kunststoff oder einem Kompositwerkstoff, ebenfalls kann der Gelenkeinsatz 1 mit einer Kunststoffschicht ummantelt sein.

Sämtliche Varianten der Figuren 1 bis 4 können mit einem Silikon oder einen anderen Elastomer umspritzt sein. Ebenfalls ist es möglich, dass statt einer Blechausgestaltung das Verbindungselement 1 aus einem elastischen Kunststoff oder einem Kompositmaterial ausgebildet ist, beispielsweise einem faserverstärkten, streifenartigen Werkstoff.

## Patentansprüche

1. Gelenkeinsatz (1) mit einem oberen Anschlussmittel (2) zur Befestigung an einem ersten orthopädischen Bauteil und einem unteren Anschlussmittel (3) zur Befestigung an einem zweiten, relativ zu dem ersten orthopädischen Bauteil verschwenkbar gelagerten orthopädischen Bauteil sowie mit einem zugkraftübertragenden Verbindungselement (4) zwischen den Aufnahmeeinrichtungen (2, 3), wobei das Verbindungselement (4) einen biegeelastischen, formstabilen Materialabschnitt (6, 6') aufweist, der einen Querschnitt mit unterschiedlichen axialen Flächenträgheitsmomenten aufweist und die Achse des kleineren axialen Flächenträgheitsmomentes zumindest abschnittsweise parallel zu der Schwenkachse der orthopädischen Bauteile liegt, **dadurch gekennzeichnet, dass** der Materialabschnitt (6, 6') in seiner Längserstreckung in sich verdreht ist.

2. Gelenkeinsatz nach Anspruch 1, **dadurch gekennzeichnet, dass** der Materialabschnitt (6, 6') um zumindest 90° in sich verdreht ist.

3. Gelenkeinsatz nach Anspruch 1 oder 2, **dadurch gekennzeichnet, dass** der Materialabschnitt (6, 6') um ganzzahlige Vielfache von 90° verdreht ist, insbesondere um 180° oder 360°.

4. Gelenkeinsatz nach einem der voranstehenden Ansprüche, **dadurch gekennzeichnet, dass** das Materialabschnitt (6, 6') aus Blech oder Kunststoff besteht.

5. Gelenkeinsatz nach einem der voranstehenden Ansprüche, **dadurch gekennzeichnet, dass** das Verbindungselement (4) zwei separate Materialabschnitte (6) mit daran angeordneten Formschlusselementen (5) aufweist, die zugkraftübertragend ineinander eingreifen und eine Verschwenkbarkeit um eine Achse (45) zulassen.

6. Gelenkeinsatz nach einem der voranstehenden Ansprüche, **dadurch gekennzeichnet, dass** der Materialabschnitt (6, 6') einlagig ausgebildet ist.

7. Gelenkeinsatz nach einem der Ansprüche 1 bis 5, **dadurch gekennzeichnet, dass** der Materialabschnitt (6, 6') aus einer geschlossenen Schlaufe ausgebildet ist.

8. Gelenkeinsatz nach einem der Ansprüche 1 bis 4, **dadurch gekennzeichnet, dass** das Verbindungselement (4) als eine geschlossene Schlaufe mit einander gegenüberliegenden Materialabschnitten (6, 6') ausgebildet ist.

9. Gelenkeinsatz nach einem der voranstehenden Ansprüche, **dadurch gekennzeichnet, dass** die Anschlussmittel (2, 3) eine Hülse (12, 13, 22, 23, 32, 33, 42, 43) aufweisen.

10. Gelenkeinsatz nach einem der voranstehenden Ansprüche, **dadurch gekennzeichnet, dass** die Anschlussmittel (2, 3) separat an dem Verbindungselement befestigt sind.

11. Gelenkeinsatz nach einem der Ansprüche 1 bis 9, **dadurch gekennzeichnet, dass** die Anschlussmittel (2, 3) einstückig mit dem Materialabschnitt als Umformabschnitt, Wicklung oder Schlaufenendabschnitt ausgebildet ist.

12. Gelenkeinsatz nach einem der voranstehenden Ansprüche, **dadurch gekennzeichnet, dass** das Verbindungselement (4) ummantelt ist, insbesondere von einem flexiblen Elastomer.

## Claims

1. An articulated insert (1) with an upper connecting means (2) for fastening to a first orthopedic component and with a lower connecting means (3) for fastening to a second orthopedic component that is mounted pivotably relative to the first orthopedic component, and with a tensile force-transmitting joining element (4) between the receiving devices (2, 3), wherein the joining element (4) has a flexurally elastic, dimensionally stable material section (6, 6'), which has a cross section with different axial area moments of inertia, and the axis of the smaller axial area moment of inertia is parallel at least in part to the pivot axis of the orthopedic components, **characterized in that** the material section (6, 6') is twisted in its longitudinal extent.

2. The articulated insert as claimed in claim 1, **characterized in that** the material section (6, 6') is twisted through at least 90°,

3. The articulated insert as claimed in claim 1 or 2, **characterized in that** the material section (6, 6') is twisted through integral multiples of 90°, in particular through 160° or 360°.

4. The articulated insert as claimed in one of the preceding claims, **characterized in that** the material section (6, 6') is made of sheet metal or plastic.

5. The articulated insert as claimed in one of the preceding claims, **characterized in that** the joining element (4) has two separate material sections (6), with form-fit elements (5) which are arranged thereon and which engage in each other in a manner transmitting tensile force and permit a pivotability about an axis (45).

6. The articulated insert as claimed in one of the preceding claims, **characterized in that** the material section (6, 6') is configured in one layer.

7. The articulated insert as claimed in one of claims 1 through 5, **characterized in that** the material section (6, 6') is formed from a closed loop.

8. The articulated insert as claimed in one of claims 1 through 4, **characterized in that** the joining element (4) is designed as a closed loop with mutually opposite material sections (6, 6').

9. The articulated insert as claimed in one of the preceding claims, **characterized in that** the connecting means (2, 3) have a sleeve (12, 13, 22, 23, 32, 33, 42, 43).

10. The articulated insert as claimed in one of the preceding claims, **characterized in that** the connecting means (2, 3) are fastened separately to the joining element.

11. The articulated insert as claimed in one of claims 1 through 9, **characterized in that** the connecting means (2, 3) is formed in one piece with the material section as a deformation section, winding or loop-end section.

12. The articulated insert as claimed in one of the preceding claims, **characterized in that** the joining element (4) is encapsulated, in particular by a flexible elastomer.

## Revendications

1. Insert articulaire (1) comprenant un moyen de raccordement supérieur (2) pour la fixation à un premier élément orthopédique et un moyen de raccordement inférieur (3) pour la fixation à un deuxième élément orthopédique monté pivotant relativement au premier élément orthopédique, ainsi que, entre les dispositifs récepteurs (2, 3), un élément de liaison (4) transmetteur de force de traction, l'élément de liaison (4) présentant un tronçon de matériau (6, 6') indéformable élastique en flexion qui présente une section avec différents moments d'inertie de surface axiaux, et l'axe du plus petit moment de surface d'inertie axial étant au moins sur une portion parallèle à l'axe de pivotement des éléments orthopédiques, , **caractérisé en ce que** le tronçon de matériau (6, 6') est vrillé sur lui-même dans sa direction d'extension longitudinale.

2. Insert articulaire selon la revendication 1, **caractérisé en ce que** le tronçon de matériau (6, 6') est vrillé d'au moins 90° sur lui-même.

3. Insert articulaire selon la revendication 1 ou 2, **caractérisé en ce que** le tronçon de matériau (6, 6') est vrillé de multiples entiers de 90°, en particulier de 180° ou 360°.

4. Insert articulaire selon l'une quelconque des revendications précédentes, **caractérisé en ce que** le tronçon de matériau (6, 6') est en tôle ou en matière synthétique.

5. Insert articulaire selon l'une quelconque des revendications précédentes, **caractérisé en ce que** l'élément de liaison (4) présente deux tronçons de matériau (6) séparés ayant des éléments de liaison par formes conjuguées (5) agencés sur ceux-ci, qui entrent en prise l'un dans l'autre de façon transmettrice de force de traction et permettent une possibilité de pivotement autour d'un axe (45).

6. Insert articulaire selon l'une quelconque des revendications précédentes, **caractérisé en ce que** le tronçon de matériau (6, 6') est formé monocouche.

7. Insert articulaire selon l'une quelconque des revendications 1 à 5, **caractérisé en ce que** le tronçon de matériau (6, 6') est formé par une boucle fermée.

8. Insert articulaire selon l'une quelconque des revendications 1 à 4, **caractérisé en ce que** l'élément de liaison (4) est formé par une boucle fermée ayant des tronçons de matériau (6, 6') se faisant face,

9. Insert articulaire selon l'une quelconque des revendications précédentes, **caractérisé en ce que** les moyens de raccordement (2, 3) présentent un manchon (12, 13, 22, 23, 32, 33, 42, 43).

10. Insert articulaire selon l'une quelconque des revendications précédentes, **caractérisé en ce que** les moyens de raccordement (2, 3) sont fixés séparément à l'élément de liaison.

11. Insert articulaire selon l'une quelconque des revendications 1 à 9, **caractérisé en ce que** les moyens de raccordement (2, 3) sont formés d'un seul tenant avec le tronçon de matériau, par un tronçon conformé, un enroulement ou un tronçon d'extrémité de boucle.

12. Insert articulaire selon l'une quelconque des revendications précédentes, **caractérisé en ce que** l'élément de liaison (4) est gainé, en particulier par un élastomère flexible.
